Europäisches Patentamt

European Patent Office

Office européen des brevets

(11) Veröffentlichungsnummer: **0 010 677**
**A1**

## (12) EUROPÄISCHE PATENTANMELDUNG

(21) Anmeldenummer: 79103969.6

(22) Anmeldetag: 15.10.79

(51) Int. Cl.³: **C 09 K 3/30**, A 61 K 7/11, A 61 K 7/32

(30) Priorität: 27.10.78 DE 2846811

(43) Veröffentlichungstag der Anmeldung: **14.05.80**
**Patentblatt 80/10**

(84) Benannte Vertragsstaaten: **BE CH DE FR GB IT NL SE**

(71) Anmelder: **BAYER Aktiengesellschaft, Zentralbereich Patente,Marken und Lizenzen Bayerwerk, D-5090 Leverkusen 1 (DE)**

(72) Erfinder: **Mitschke, Karl-Heinz, Dr., Am Berg 22, D-5068 Odenthal (DE)**
Erfinder: **Niederprüm, Hans, Dr., Hofstrasse 27, D-4019 Monheim (DE)**

(54) **Treibgas für Spraydosen, seine Verwendung und dieses Gas enthaltende Spraydosen.**

(57) Die vorliegende Erfindung betrifft eine Treibgasmischung für Spraydosen, die als einen wesentlichen Bestandteil 1,1-Difluor-2-chloräthylen enthält.

EP 0 010 677 A1

- 1 -

**BAYER AKTIENGESELLSCHAFT**  5090 Leverkusen, Bayerwerk
Zentralbereich  Br-mo
Patente, Marken und Lizenzen

BEZEICHNUNG GEÄNDERT
siehe Titelseite

Treibgasmischung für Spraydosen

Die vorliegende Erfindung betrifft ein neues Treibgas
für Spraydosen, das eine feinste Vernebelung des Wirkstoff-Lösungsmittelgemisches gestattet.

Die wichtigsten Treibgase für Aerosoldosen sind bekanntlich die perhalogenierten Fluorchloralkane $CCl_3F$ (R 11),
$CCl_2F_2$ (R 12) und $CClF_2$-$CClF_2$ (R 114). In jüngster Zeit
ist jedoch ein erhebliches Interesse daran entstanden,
diese gebräuchlichen Treibgase durch solche mit anderen
Eigenschaften, z.B. mit besserer Abbaubarkeit, zu ersetzen. Diese neuen Treibgase sollen sonst natürlich
ähnlich günstige Eigenschaften wie die konventionellen
Fluorchloralkane besitzen. Die daraus zwangsläufig resultierende verringerte chemische Stabilität darf aber
andererseits nicht zu physiologisch bedenklichen Produkten führen, da ja die Treibgase auch für die mengen-

Le A 19 216 -Ausland

mäßig bedeutenderen Sektoren der Haar- und Körpersprays eingesetzt werden sollen.

Neben den nicht brennbaren und darüber hinaus flammhemmenden Eigenschaften besitzenden Fluorchloralkanen wurden in der Aerosoltechnik auch bereits andere Treibgase eingesetzt, z.B. Kohlenwasserstoffe wie Propan, Butan, Isobutan oder komprimierte Gase wie $N_2$, $N_2O$ oder $CO_2$ (vgl. z.B. I.I. Sciarra und L. Stoller, The Science und Technology of Aerosol Packaging, John Wiley, New York, 1974 und The Aerosol Handbook, Wayne E. Dorland Co., Caldwell, New Jersey (1972)).

Kohlenwasserstoffe besitzen jedoch den großen Nachteil der leichten Brennbarkeit und können explosible Treibgas-Luftgemische bilden. Dies ist nicht nur für den Benutzer einer Spraydose gefährlich, sondern erschwert auch das Abfüllen, den Transport und die Lagerung. Speziell bei den Abfüllstationen müssen beim Einsatz von Kohlenwasserstoffen strenge Sicherheitsvorschriften beachtet werden.

Komprimierte Gase, wie z.B. Stickstoff oder Luft sind praktisch nicht geeignet, echte Aerosole zu erzeugen. Auch die komprimierten Gase, die noch eine begrenzte Löslichkeit in den verwendeten Lösungsmitteln besitzen - wie $CO_2$ oder $N_2O$ erlauben es nicht, eine druckkonstante Aerosolpackung herzustellen, d.h. die Sprayparameter ändern sich laufend mit dem Verbrauch der flüssigen Füllung. Außerdem müssen z.B. bei der Verwendung von $CO_2$ oder $N_2O$ sehr große Mengen brennbarer Lösungsmittel, wie Aceton oder Äthanol, verwendet werden.

Es wurde auch schon vorgeschlagen, die technischen Produkte $CHF_2Cl$ (R 22) - ein Tieftemperatur-Kältemittel bzw. PTFE-Vorprodukt - und $CF_2Cl-CH_3$(R 142 b) - ein Vorprodukt für Fluorkunststoffe und -elastomere- bzw. Mischungen hieraus als Treibgase einzusetzen (M. Bhuta, Aerosol Age, Dec. 1976, Seite 45). R 142 b jedoch ist brennbar (Explosionsgrenze in Luft 9,0/14,8 %) und R 22 wegen seines tiefen Siedepunktes von -40,8°C und damit des hohen Dampfdrucks bei Raumtemperatur nur bedingt allein einsetzbar.

Auch 1,1,1-Trifluor-2-chloräthan (R 133 a) wurde schon als Treibgas eingesetzt. So wurde R 133 a für gewisse Aerosolformulierungen vorgeschlagen, wobei z.B. die gegenüber konventionellen Fluorchlorkohlenwasserstoffen erhöhte Wasserlöslichkeit für Rasiercremes ausgenutzt wurde (vgl. DT-AS 1 542 076).

Wegen des relativ hohen Siedepunktes von 1,1,1,-Trifluor-2-chloräthan (ca. 7°C)ist es erforderlich, zusätzlich sog. "Niedrigsieder" beizumischen, wie etwa der übliche Fluorchlorkohlenwasserstoff R 12 (US-Pat. 3583921) oder Kohlenwasserstoffe, wie etwa Propan oder/und Butan, Kohlendioxid oder/und Distickstoffoxid, um den Anforderungen für ein echtes Aerosol-Produkt zu entsprechen.

Gegenstand der vorliegenden Erfindung ist ein Treibgas für Spraydosen, welches dadurch gekennzeichnet ist, daß es 1,1-Difluor-2-chloräthylen enthält.

Des weiteren ist die Verwendung von 1,1-Difluor-2-chlor-äthylen als Treibgas Gegenstand der Erfindung.

Le A 19 216

1,1-Difluor-2-chloräthylen, eine an sich bekannte Verbindung ohne bislang nennenswerte technische Bedeutung, lässt sich nach einem bisher unveröffentlichten Vorschlag durch Dehydrohalogenierung von 1,1-Difluor-1,2-dichloräthan mit wässrigen Basen unter Druck und erhöhter Temperatur ohne Zusätze wie organische Lösungsmittel oder Emulgatoren in guten Ausbeuten herstellen. In einer allgemeinen Ausführung wird so verfahren, daß in einem Druckbehälter, der mit den üblichen Zusätzen, wie z. B. Rührer, Druckkühler, Druckkonstanthalteventile usw. versehen ist, eine der Reaktionskomponenten vorgelegt und die andere, vorzugsweise der basisch wirkende Stoff, in Wasser gelöst oder suspendiert, zudosiert wird. Das während der Reaktion entstehende 1,1-Difluor-2-chloräthylen wird kontinuierlich über das Druckkonstanthalteventil in auf ca.-70°C gekühlte Fallen kondensiert und nach dem Trocknen (z. B. mit Calciumchlorid) destilliert. Die Destillation kann dabei unter Druck oder bei Normaldruck erfolgen. Eventuell im Sumpf verbleibendes Ausgangsmaterial kann gegebenenfalls erneut zur Reaktion gebracht werden. 1,1-Difluor-1,2-dichloräthan wiederum erhält man durch katalytische Flüssigphasen-Fluorierung von Trichloräthylen.

1,1-Difluor-2-chloräthylen ist aufgrund seines chemischen Aufbaus angreifbar, d.h. abbaubar und somit als Substitutionsprodukt für die herkömmlichen Fluorchlorkohlenwasserstoff-Typen in Aerosol-Formulierungen zu betrachen. Beim Einsprühen in eine Bunsenbrennerflamme wird keine stationäre Verbrennung beobachtet. Erst in einer geschlossenen Apparatur bei Anwendung sehr hoher Zündenergie (ca. 20 Joule) wurden untere und obere Zündgrenzen von 10,1 Vol.-% und 41,3 Vol.-% in Luft beobachtet. Es besitzt gute Lösungseigenschaften und zeigt keinen nachteiligen Einfluß auf die Parfümierung von Aerosol-Packungen. Aus dem

Le A 19 216

- 5 -

günstigen Siedepunkt von ca. -18°C resultiert bei 20°C ein Druck von ca. 3,5 bar, der etwa dem der 50/50-Mischung aus den herkömmlichen Fluorchlorkohlenwasserstoffen R 11 und R 12 entspricht, so daß keine zusätzlichen "Druckverstärker" bzw. "Druckreduzierer" zur Erzielung guter Sprayeigenschaften notwendig sind.

Als Lösungsmittel für die Wirksubstanzen können z.B. Äthanol, Äthylacetat, Isopropanol, Aceton, Methylchloroform oder Methylenchlorid oder Gemische dieser Lösungsmittel zugesetzt werden. Ferner ist es auch möglich, fluorhaltige Lösungsmittel, wie z.B. $CF_2Cl-CFCl_2$ oder $CF_2Cl-CH_2Cl$ einzusetzen.

Die Menge an Treibgas wird in an sich bekannter Weise entsprechend dem Anwendungszweck der Aerosoldose zugegeben. Im allgemeinen beträgt der Treibgasanteil 8 - 92, teilweise sogar 96 Gew.-%, (bezogen auf den Gesamtinhalt der Sprühdose), je nachdem ob ein Schaum (niederer Treibgasanteil), ein Oberflächenspray (mittlerer Treibgasanteil) oder ein echtes Aerosol (hoher Treibgasanteil) gewünscht wird.

Das erfindungsgemäße Treibgas lässt sich für alle Arten von Spray-Systemen einsetzen. Beispielhaft seien die Haarsprays, Körpersprays, Raum- und Insektizidsprays,

Le A 19 216

Polituren, Lacke, Sonnenschutzsprays, Rasierschäume,
Shampoos, Reinigungsschäume, Kfz-Pflegemittel,
medizinische Sprays, Gewürzsprays oder Siliconsprays
genannt.

Der Gegenstand der vorliegenden Erfindung soll anhand
der folgenden Beispiele noch näher erläutert werden.

### Beispiel 1

### <u>Aerosol-Körperspray</u>

0,5 Gew.-% 2,4,4'-Trichlor-2'-hydroxydiphenyläther, 1,0 Gew.-% Triglyceridgemisch von gesättigten Pflanzenfettsäuren mittlerer Kettenlänge und 1,0 Gew.-% eines handelsüblichen Parfümöls wurden in 32,95 Gew.-% Äthanol gelöst. Dieses Füllgut wurde in eine 6 oz. Spraydose überführt und mit 65,0 Gew.-% des Treibmittels 1,1-Difluor-2-chloräthylen aufgefüllt. Die Dose wurde mit einem handelsüblichen Ventil (z.B. Co-10/goldl. 0,3) verclincht; als Sprühkopf diente eine ebenfalls handelsübliche Vorrichtung.

Eine anschließende Druckmessung ergab bei 20°C einen Druck von 3,3 bar. Die Sprühcharakteristik entsprach, wie auch bei den folgenden Beispielen, der einer mit herkömmlichen Treibmitteln gefüllten Sprühdose.

### Beispiel 2
### <u>Aerosol Haarspray</u>

Eine Mischung bestehend aus 5,0 Gew.-% Polyvinylpyrolidon/ Vinylacetat (30 : 70), 14,8 Gew.-% Methylenchlorid, 20,0 Gew.-% Isopropanol und 0,2 Gew.-% eines handelsüblichen Parfümöls wurden mit 60 Gew.-% des Treibmittels 1,1 -Difluor-2-chloräthylen in eine 6 oz. Sprühdose überführt und wie in Beispiel 1 verfahren. Die Druck-

- 8 -

messung bei 20°C ergab einen Druck von 3,0 bar. Die Abfüllung entsprach den Eigenschaften eines handelsüblichen
Produktes.

Beispiel 3
Aerosol-Raumspray

19,0 Gew.-% Äthanol, 1,0 Gew.-% eines handelsüblichen
Parfümöls und 80,0 Gew.-% des Treibmittels 1,1-Difluor-
2-chloräthylen wurden in eine 6 oz. Sprühdose gefüllt
und wie in Beispiel 1 weiterverfahren. Eine anschließende
Druckprüfung ergab bei 20°C einen Druck von 3,5 bar.

Beispiel 4
Aerosol-Rasierschaum

6,8 Gew.-% Stearinsäure, 3,7 Gew.-% Triäthanolamin,
0,5 Gew.-% Polyäthylenglykol MG 1550, 0,5 Gew.-% Alkanolamid, 0,25 Gew.-% Polyäthylenglykol 6000, 2,0 Gew.-%
Glycerin, 1,0 Gew.-% Propylenglykolstearat und 84,25 Gew.-%
dest. Wasser wurden zusammen auf ca. 70°C erwärmt und
gut verrührt. Nach dem Abkühlen auf ca. 40°C wurde die
Mischung mit 1,0 Gew.-% eines handelsüblichen Parfüms
unter Rühren versetzt.

90,0 Gew.-% dieses Füllgutes wurden mit 10,0 Gew.-% des
Treibmittels 1,1-Difluor-2-chloräthylen in eine 6 oz.
Sprühdose gefüllt und mit einem handelüblichen Ventil und
Schaumkopf versehen. Nach dem Schütteln der Dose wurde
ein äußerst feinverteilter Schaum erhalten.

Le A 19 216

Beispiel 5

Aerosol-Insektizidspray

20 Gew.-%Füllgut (handelsübliches Insektizid basierend auf DDVP und synth. Pyrethrum) und 80 Gew.-% des Treibmittels 1,1-Difluor-2-chloräthylen wurden in eine 6 oz. Sprühdose gefüllt und wie in Beispiel 1 verschlossen. Die Abfüllung entsprach den Eigenschaften eines handelsüblichen Produktes.

Beispiel 6

Aerosol-Siliconspray

4 Gew.-% eines Polydimethylsiloxan-Öls der Viskosität 300 cSt wurden mit 96 Gew.-% des Treibmittels 1,1-Difluor-2-chloräthylen in eine 6 oz. Sprühdose gefüllt und wie in Beispiel 1 verschlossen. Die Abfüllung entsprach den Eigenschaften eines handelsüblichen Produktes.

Le A 19 216

- 10 -

## Patentansprüche

1) Treibgas für Spraydosen, dadurch gekennzeichnet,
daß es 1,1-Difluor-2-chloräthylen enthält.

2) Verwendung von 1,1-Difluor-2-chloräthylen als
Treibgas.

3) Spraydose, enthaltend neben dem üblichen Füllgut
1,1-Difluor-2-chloräthylen.

<u>Le A 19 216</u>

**Europäisches Patentamt**

**EUROPÄISCHER RECHERCHENBERICHT**

0010677

Nummer der Anmeldung

EP 79 10 3969

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | FR - A - 1 174 825 (C.F. BOE)<br>* Seite 3, linke Spalte, Zeilen 7-15 *<br><br>---- | 1-3 |

**KLASSIFIKATION DER ANMELDUNG (Int.Cl. 3)**

C 09 K 3/30
A 61 K 7/11
7/32

**RECHERCHIERTE SACHGEBIETE (Int. Cl. 3)**

C 09 K 3/30

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 18-12-1979 | BOULON |

EPA form 1503.1 06.78